# FASCICULE DE BREVET EUROPEEN

(11) **EP 2 817 622 B1**
(45) Date de publication et mention de la délivrance du brevet: **03.06.2020**
(21) Numéro de dépôt: 13720022.6
(22) Date de dépôt: 21.02.2013
(51) Int. Cl.: G01N 33/543, G01N 33/00

(54) **CAPTEURS DE NEZ OU DE LANGUE ÉLECTRONIQUE**
ELEKTRONISCHE NASEN- ODER ZUNGENSENSOREN
ELECTRONIC NOSE OR TONGUE SENSORS

(30) Priorité: 21.02.2012 FR 1251579
(43) Date de publication de la demande: 31.12.2014
(73) Titulaire: Commissariat à l'Énergie Atomique et aux Énergies Alternatives, 75015 Paris (FR); Université Paris Sud (UPS), 91405 Orsay Cedex (FR)
(72) Inventeur: LIVACHE, Thierry, F-38560 Jarrie (FR); BUHOT, Arnaud, F-38960 Saint Etienne de Crossey (FR); BONNAFFE, David, F-75011 Paris (FR); HOU-BROUTIN, Yanxia, F-38850 Bilieu (FR)
(74) Mandataire: Gevers & Orès
(86) Numéro de dépôt international: PCT/IB2013/051421
(87) Numéro de publication internationale: WO 2013/124810

(56) Documents cités:
- EP-A1- 0 878 711
- WO-A1-2009/033370
- WO-A2-03/045862
- WO-A2-2005/118870
- US-A1- 2002 076 804
- US-A1- 2005 233 459
- US-A1- 2010 222 224
- US-B1- 6 730 201
- ALIZADEH ET AL: "Electronic nose based on the polymer coated SAW sensors array for the warfare agent simulants classification", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 129, no. 1, 17 January 2008 (2008-01-17), pages 412-423, XP022424896, ISSN: 0925-4005, DOI: 10.1016/J.SNB.2007.08.044
- Kea-Tiong Tang ET AL: "An Electronic-Nose Sensor Node Based on a Polymer-Coated Surface Acoustic Wave Array for Wireless Sensor Network Applications", Sensors, vol. 11, no. 12, 28 April 2011 (2011-04-28), pages 4609-4621, XP055443780, DOI: 10.3390/s110504609

## Description

### Domaine de l'invention

La présente invention concerne un capteur utile pour la fabrication de nez ou de langues électroniques, et son utilisation pour analyser des échantillons fluides, et en particulier des échantillons liquides ou gazeux.

### Arrière-plan technique

Les biocapteurs peuvent être séparés en deux familles distinctes.

La première famille implique une analyse grâce à une, ou des, interaction(s) spécifique(s). On peut citer, à titre d'exemple, le cas des biocapteurs à glucose utilisant une enzyme ou des systèmes multiparamétriques (plusieurs zones sensibles) de type puce à ADN ou à protéines, qui reconnaissent des ligands par liaisons spécifiques et sélectives (ADN/ADN ou antigène/protéine...). Ces biopuces sont très utilisées et génèrent une information pertinente par zone sensible composée d'un seul récepteur.

La deuxième famille de biocapteurs comprend des systèmes dits « non-spécifiques ». Dans ce cas, l'information générée par une seule zone sensible, portant un seul récepteur n'est pas interprétable car les interactions entre les composés du milieu à analyser et le récepteur ne sont pas connues. De plus, du fait de la faible spécificité de ce type de capteur, des réactions croisées entre les différents récepteurs peuvent également être observées. On utilise donc un ensemble de récepteurs générant chacun une information : l'ensemble de ces informations génère ainsi un motif (« *pattern* ») comparable à une empreinte digitale. Pour les besoins de l'analyse, ces motifs peuvent être comparés à ceux issus d'un apprentissage préalable. Cette similarité vis-à-vis des procédés utilisés par la nature à conduit à appeler ces capteurs « nez électronique » pour ce qui concerne l'étude de milieux gazeux ou « langue électronique » pour l'étude des milieux liquides.

Ces nez ou langues électroniques sont donc composés d'un ensemble de zones sensibles indépendantes, chacune portant un revêtement composé d'un récepteur apte à interagir avec le milieu étudié. Chaque zone sensible doit donc être associée à un système permettant de quantifier une interaction, généralement en mesurant l'intensité d'un signal généré par l'interaction. Dans tous les cas existant actuellement, chaque zone sensible génère une information indépendante de celle de son voisin. Ainsi, une matrice de *n* zones sensibles, générera *n* informations indépendantes qui construiront le motif d'interaction, souvent représenté par un histogramme, le numéro de la zone sensible étant associée à une valeur d'intensité de signal mesurée sur celle-ci.

Généralement, ces zones sensibles sont fabriquées de telle sorte qu'elles aient des affinités ou, plus généralement, des propriétés physico-chimiques variables vis-à-vis des milieux analysés (Turner & Magan (2004) Nature Reviews Microbiology 2:161-166). Plus récemment, des approches combinatoires ont vu le jour et des récepteurs peptidiques ont été greffés sur les zones sensibles. Cette méthode permet de générer facilement un grand nombre de récepteurs différents (Edwards et al. (2007) J. Am. Chem. Soc. 129:13575-13583). Chaque zone sensible contient un et un seul type de peptide et les informations générées restent indépendantes. Les représentations sont d'ailleurs montrées sous forme de nuages de points.

Dans ce type d'analyse, chaque information a donc la même valeur et une pondération n'est pas facile à réaliser. Ces capteurs ne fournissent donc pas une réponse continue. Or, il s'avère que dans ces domaines de langue ou de nez électronique, la réalisation des objets reste complexe et un défaut sur l'une des zones sensibles reste très fréquent. La réponse de ce capteur sera donc faussée et si aucune pondération n'est possible (les zones sensibles sont indépendantes les unes des autres), le motif généré sera difficile à interpréter.

Par ailleurs, des capteurs de type nez électronique générant des signaux continus grâce à une lecture optique ont été décrit par Di Natale et al. (2009) Sensors & Actuators B 142:412-7. Ces capteurs comprennent une couche de colorants sensibles recouverte par des polymères perméables. La réaction des colorants après diffusion de composés gazeux à analyser dans le polymère est alors enregistrée à l'aide d'une caméra. La continuité de la réponse est apportée dans ce cas par les propriétés de la couche diffusante et non par les récepteurs eux-mêmes. Ce procédé reste limité par la capacité de réaction des colorants en réponse aux composés gazeux. Le procédé n'est donc pas applicable à l'analyse de molécules complexes ne pouvant pas diffuser à travers les polymères. D'autre part, la couche de colorants sensibles étant emprisonnée sous une couche de diffusion, à savoir les polymères, aucune interaction directe entre les récepteurs du capteur et l'échantillon ne peut avoir lieu puisqu'une barrière polymère est nécessaire au fonctionnement du capteur.

Il reste donc à fournir des capteurs de type nez ou langue électronique d'application plus générale, notamment capables de générer une réponse continue.

### Description de l'invention

La présente invention découle notamment de la mise en évidence inattendue, par les inventeurs, que des capteurs pour langue ou nez électronique, comprenant un support sur plusieurs portions, ou zones sensibles, duquel sont fixés des mélanges de deux récepteurs différents, en des rapports différents, présentent un profil d'émission de signaux, générés par l'interaction de constituants d'un échantillon mis en contact avec les récepteurs du capteur, par les différentes zones sensibles, qui est non linéaire et suffisamment complexe pour être caractéristique d'un composé donné.

La non-linéarité du signal indique que la quantité d'informations obtenue est supérieure à celle que l'on obtiendrait à partir des zones sensibles composées des récepteurs purs.

Entre outre, l'augmentation du nombre de zones sensibles permet de tendre vers un profil d'émission de signaux essentiellement continu, ce qui permet avantageusement de détecter et d'éliminer d'éventuelles zones sensibles défectueuses.

Enfin, l'utilisation de mélanges d'un faible nombre de récepteurs différents permet de générer un grand nombre de zones sensibles différentes, ce qui est avantageux sur le plan économique, dans la mesure où cela limite les coûts liés au développement de récepteurs différents.

La présente invention concerne ainsi un capteur pour langue ou nez électronique pour analyser un échantillon ou détecter au moins une cible, comprenant un support sur une surface duquel se trouve une pluralité de zones sensibles comprenant chacune au moins un récepteur, chaque zone sensible pouvant émettre un signal mesurable généré par l'interaction d'au moins un constituant de l'échantillon ou d'au moins une cible avec au moins un récepteur, caractérisé en ce qu'il comprend au moins trois zones sensibles qui diffèrent entre elles par leurs compositions respectives en récepteur, l'une au moins des zones sensibles comprenant un mélange d'au moins deux récepteurs différents, ou non identiques, les deux autres zones sensibles comprenant chacune au moins un des deux récepteurs.

Comme cela apparaîtra clairement à l'homme du métier, le capteur selon l'invention est notamment utile pour mettre en œuvre une langue électronique, en particulier dans le cadre de l'analyse d'échantillons de milieux liquide, ou un nez électronique, en particulier dans le cadre de l'analyse d'échantillons de milieux gazeux.

Ainsi, la présente invention concerne également l'utilisation d'un capteur tel que défini ci-dessus pour analyser un échantillon.

La présente invention concerne également un procédé pour analyser un échantillon, dans lequel :
- on met en contact un capteur tel que défini ci-dessus avec l'échantillon ;
- on mesure les signaux émis par les zones sensibles du capteur ;
- on compare les signaux mesurés à l'étape précédente aux signaux générés indépendamment par les zones sensibles du même capteur, ou d'un deuxième capteur similaire, consécutivement à une mise en contact avec au moins un autre échantillon ;
- on qualifie l'échantillon analysé.

La présente invention concerne également l'utilisation d'un capteur tel que défini ci-dessus pour définir la composition en récepteurs d'un mélange de récepteurs pour revêtir une structure tridimensionnelle destinée à interagir ou à ne pas interagir avec au moins une cible

La présente invention concerne également un procédé de préparation d'une structure tridimensionnelle revêtue d'un mélange de récepteurs destinée à interagir ou à ne pas interagir avec au moins une cible, comprenant :
- la mise en contact de la cible avec un capteur tel que défini ci-dessus ;
- l'identification d'une zone sensible du capteur émettant un signal dont l'intensité est indicative d'une affinité avec la cible, qui peut être forte ou faible, similaire à l'affinité souhaitée de la structure tridimensionnelle revêtue d'un mélange de récepteurs pour la cible ;
- éventuellement répéter ces deux étapes pour d'autres cibles si nécessaire ;
- revêtir la structure tridimensionnelle avec un mélange de récepteurs ayant une composition en récepteurs similaire à celle de la zone sensible identifiée.

### Récepteur

Comme on l'entend ici, « un récepteur » est un composé, qui peut être de tout type chimique, susceptible d'interagir, par lui-même ou lorsqu'il est assemblé avec un ou plusieurs autres récepteurs au sein d'un mélange pour former un assemblage de récepteurs, avec un ou plusieurs constituants de l'échantillon ou avec une cible. Comme on l'entend ici, un récepteur peut également être désigné « élément ou déterminant de reconnaissance ou de liaison avec un ou plusieurs constituants de l'échantillon ou avec une cible ».

De préférence, les récepteurs selon l'invention diffèrent entre eux selon aux moins une caractéristique physico-chimique, telle que l'hydrophilie ou l'hydrophobicité d'une part et la densité électronique, la polarité, ou la charge d'autre part. Par ailleurs, d'autres caractéristiques de conformation ou de configuration peuvent les différencier : ainsi les récepteurs peuvent différer entre eux selon la présence de structures secondaires, telles que des hélices ou des feuillets, ou bien être dans des relations de stéréoisomérie entre eux.

Ainsi, sur le plan structural, les récepteurs selon l'invention peuvent être :
- des molécules simples, notamment de 1000 Da ou moins, telles que des ions, des complexes métalliques, des composés organiques, notamment des composés organométalliques, des acides aminés, des peptides, des oses, des oligosides, des nucléotides, des oligonucléotides ; ou
- des molécules complexes, notamment de plus de 1000 Da, telles que des polypeptides ou des protéines, éventuellement glycosylé, des polyosides, des lipides, des ADN, des ARN, ou des polymères organiques.

Les récepteurs selon l'invention peuvent également constituer des « briques » moléculaires dont l'assemblage au sein d'un mélange, notamment de type combinatoire, aboutit à une construction moléculaire interagissant avec un ou plusieurs constituants de l'échantillon ou avec une cible. Des assemblages de récepteurs de ce type sont notamment décrits dans Ojeda et al. (2007) Carbohydrate Research 342:448-459 ; Di Gianvincenzo et al. (2010) Bioorganic & Medicinal Chemistry Letters 20:2718-2721 ; Bresee et al. (2010) Chem. Commun. 46:7516-7518 ; Bresee et al. (2011) Small 7:2027-2031 ; ou encore Wolfenden & Cloninger, (2006) Bioconjugate Chem. 17:958-966.

De préférence, les récepteurs selon l'invention ont une taille inférieure à 1 000 000 Da.

Comme on l'entend ici, les récepteurs selon l'invention peuvent être indépendants entre eux ou bien être reliés entre eux par des liens moléculaires, notamment covalents. Lorsque les récepteurs sont reliés entre eux, leur association peut former une macromolécule présentant différents sites d'interaction avec les constituants de l'échantillon, ces sites correspondant aux récepteurs selon l'invention.

### Zone sensible

Une « zone sensible » selon l'invention est une portion du support susceptible d'émettre un signal généré par l'interaction entre un ou plusieurs constituants de l'échantillon avec les récepteurs qu'elle comprend.

Une telle zone sensible occupe une surface ou un volume sur le support qui peut être de topologie et de forme variables. Ainsi, elle peut être plane ou en relief, c'est-à-dire que les récepteurs sont fixés directement sur le support ou occupent un volume situé au dessus de la surface en se répartissant ainsi dans les trois dimensions. Par ailleurs, la zone sensible selon l'invention peut adopter une forme quelconque, elle peut notamment être circulaire ou polygonale, en particulier parallélépipédique. La zone sensible selon l'invention peut en outre être séparée ou accolée d'au moins une autre zone sensible. De préférence, l'ensemble des zones sensibles d'un capteur ont la même surface, la même topologie et la même forme. De manière particulièrement préférée, les zones sensibles selon l'invention ont une topologie plane et une forme circulaire. De manière préférée également, la surface d'une zone sensible selon l'invention est d'environ 0,25 µm² à 10 mm². En outre, de manière préférée l'épaisseur de la zone sensible sera comprise entre 0,5 nm et 100 µm.

La mise en contact d'un échantillon avec un capteur selon l'invention provoque l'émission de plusieurs signaux mesurables, au moins un signal pour chacune des zones sensibles, formant ainsi un profil de signaux, qui est caractéristique de l'échantillon. Comme l'homme du métier le comprendra bien, l'augmentation du nombre de zones sensibles permet donc d'augmenter la résolution des profils de signaux mesurables.

Ainsi, de préférence, le capteur selon l'invention peut comprendre au moins 3, 4, 5, 6, 7, 8, 9, 10, 15, 20, 25, 50, 100, 500, 1000, 10 000, 100 000, ou 1 000 000 zones sensibles selon l'invention. De préférence également, le capteur selon l'invention peut comprendre moins de 10 000 000, 1.000 000, 100000, 10000, 5 000, 1000, 500, 100, 50, ou 25 zones sensibles selon l'invention.

La densité surfacique des récepteurs permet de moduler l'intensité du signal mesurable.

La densité surfacique d'un récepteur au sein d'une zone sensible est donnée par le nombre de récepteurs compris dans une zone sensible rapporté à la surface que cette même zone sensible occupe sur le support. Lorsque plusieurs récepteurs non identiques sont présents dans une zone sensible, il est possible de définir les densités surfaciques respectives de chacun des récepteurs non identiques ainsi que la densité surfacique de l'ensemble des récepteurs. La densité surfacique d'un récepteur peut être uniforme dans l'ensemble de la zone sensible ou être variable. Lorsqu'elle est variable, les récepteurs peuvent être répartis selon des gradients de densité surfaciques, par exemple en fonction de directions dans le plan défini par la surface du support. De préférence, la densité surfacique de l'ensemble des récepteurs au sein d'une zone sensible est comprise entre 10⁸ à 10¹⁵ récepteurs/mm². De préférence, l'ensemble des zones sensibles du capteur ont des densités surfaciques de l'ensemble des récepteurs qu'elles comprennent qui sont uniformes et essentiellement similaires.

Par ailleurs, l'augmentation du nombre de zones sensibles non identiques permet également d'augmenter la sensibilité du capteur en multipliant les interactions possibles entre les constituants de l'échantillon et les zones sensibles, cela permet également d'augmenter la diversité des profils de signaux, et donc de caractériser un plus grand nombre de constituants différents au sein de l'échantillon à analyser. En outre l'augmentation du nombre de zones sensibles non identiques permet d'augmenter la continuité du profil des signaux émis par les zones sensibles du capteur, ce qui permet de repérer et d'éliminer les zones sensibles défectueuses générant des informations non pertinentes.

De préférence, le capteur selon l'invention comprend au moins 2, 3, 4, 5, 6, 7, 8, 9, 10, 15, 20, 25, 50, ou 100 récepteurs différents ou non identiques, que l'on peut également qualifier de types de récepteurs différents ou de récepteurs de nature différente. De préférence également, le capteur selon l'invention comprend au plus 1000, 100, 50, 25, ou 10 récepteurs différents ou non identiques. Une même zone sensible selon l'invention peut comprendre un seul récepteur ou entre 2 et 20 récepteurs différents ou non identiques.

Comme l'homme du métier le comprendra bien chaque récepteur, ou type de récepteur, est de préférence présent en plusieurs exemplaires dans une zone sensible selon l'invention. Ainsi, à titre d'exemple, lorsqu'une zone sensible est dite comprendre deux récepteurs différents, elle comprend de préférence plusieurs exemplaires de chacun des récepteurs différents, notamment avec les densités surfaciques définies précédemment. De même, lorsqu'on indique qu'une zone sensible ne comprend qu'un seul type de récepteur, cela signifie qu'elle peut comprendre plusieurs exemplaires d'un seul récepteur ou type de récepteur.

De préférence, dans le capteur selon l'invention, l'une au moins des zones sensibles comprend au moins 3, 4, 5, 6, 7, 8, 9 ou 10 récepteurs différents ou non identiques.

Par ailleurs, dans un mode de réalisation préféré selon l'invention, une pluralité des zones sensibles du capteur, notamment au moins 3, 5, 10 ou 100 zones sensibles, comprennent chacune des mélanges d'au moins 2, 3, 4, 5, 6, 7, 8, 9 ou 10 récepteurs différents ou non identiques, les récepteurs différents ou non identiques étant les mêmes dans chaque mélange, et diffèrent entre elles par les proportions respectives des récepteurs différents ou non identiques qu'elles comprennent. Autrement dit, le capteur comprend une pluralité de zones sensibles, notamment au moins 3, 5, 10 ou 100, qui ont chacune des proportions différentes d'au moins 2, 3, 4, 5, 6, 7, 8, 9 ou 10 récepteurs non identiques qui sont présents dans chacune des zones sensibles de la pluralité de zones sensibles.

Ainsi, dans un mode de réalisation préféré de l'invention, le capteur selon l'invention comprend n récepteurs non identiques (n nombre entier supérieur à un) et les zones sensibles comprennent des mélanges de ces n récepteurs non identiques dans des proportions variables.

Dans le cas particulier où n = 2, on peut par exemple avoir 6 zones sensibles différentes, ayant des densités surfaciques de l'ensemble des récepteurs qu'elles comprennent uniformes et égales entre elles, avec les proportions suivantes des deux récepteurs A et B considérés : A 0% B 100% ; A 20% B 80% ; A 40% B 60% ; A 60% B 40% ; A 80% B 20% ; A 100% B 0%. Le pourcentage étant ici exprimé comme la quantité du récepteur considéré dans une zone sensible sur la quantité totale de récepteurs de la zone sensible. L'augmentation de la proportion du récepteur A, de 20% en 20%, est nommée incrément. Dans ce cas l'incrément est constant, mais on peut également envisager des incréments non constants par exemple de 0, 10, 20, 30, 50, 70, 80, 90, 100% de A, complété à 100% par B.

Plus généralement, on peut envisager selon l'invention k zones sensibles au sein desquels la proportion en pourcentage d'un premier récepteur dans un mélange de deux récepteurs évolue par incrément de 100/(k-1), la densité surfacique de l'ensemble des récepteurs étant de préférence identique pour chaque zone sensible.

Ainsi, dans le mode de réalisation préféré de l'invention, selon lequel le capteur selon l'invention comprend n récepteurs non identiques (n nombre entier supérieur à un) et les zones sensibles comprennent des mélanges de ces n récepteurs non identiques dans des proportions variables, un nombre (k) préféré de zones sensibles à prévoir en fonction de l'incrément i (exprimé en pourcentage) est k = ((100/i)+n-1)!/((n-1)!(100/i)!) lorsque 100/i est un entier et n<(100/i)+1, et k = n^(100/i)/(100/i)! lorsque n>(100/i)+1.

La diminution de la valeur de l'incrément augmente la continuité et la résolution du profil de signaux. En outre, cela permet d'obtenir des signaux très proches les uns des autres, voire partiellement redondant, de ce fait de corriger, ou de pondérer, les éventuelles zones sensibles défectueuses. De préférence, l'incrément sera de 50 %, 33 %, 20 %, 10 %, 5 %, 4 %,3 %, 2 %, 1 %, 0,5 %, 0,4 %, 0,3 %, 0,2 % ou 0,1 %.

La détection de zones sensibles défectueuses est possible, notamment par comparaison des signaux émis par une zone sensible dont on souhaite évaluer la fonctionnalité avec ceux émis par des zones sensibles proches en composition. Ainsi, dans un mode de réalisation particulier des procédés selon l'invention, ces procédés comprennent en outre une étape de vérification de la fonctionnalité des zones sensibles du capteur, c'est-à-dire de leur non défectuosité, par comparaison du signal émis par une zone sensible avec celui émis par au moins deux autres zones sensibles, notamment des zones sensibles proches en composition de celle dont on souhaite vérifier la fonctionnalité. A titre d'exemple, les zones sensibles proches en composition au sens de l'invention d'une zone sensible dont on souhaite évaluer la fonctionnalité seront celles dont les pourcentages respectifs des quantités des différents récepteurs présents par zone sensible rapporté à la quantité totale des récepteurs de la zone sensible, diffèrent de préférence de moins de 50 %, 33 %, 20 %, 10 %, 5 %, 2 %, 1 %, 0,5 %, 0,2 % ou 0,1 % de ceux de la zone sensible dont on souhaite évaluer la fonctionnalité. De préférence, une zone sensible 1 sera considérée défectueuse si le signal R1 émis par cette zone sensible diffère sensiblement des signaux R2 et R3 émis par deux zones sensibles proches en composition. Par exemple, la zone sera considérée défectueuse si la différence en valeur absolue minimum de (|R1-R2|,|R1-R3|) est supérieure d'au moins 50, 20, 10, 5, 4, 3 ou 2 fois la différence |R2 -R3| entre les deux signaux R2 et R3. Dans un autre exemple, il sera possible d'utiliser la différence relative ; dans ce cas, la zone sensible 1 sera considérée défectueuse si le minimum de (2|R1-R2|/(R1+R2),2|R1-R3|/(R1+R3)) est supérieure d'au moins 50, 20, 10, 5, 4, 3, 2 fois la différence relative 2|R2-R3|/(R2+R3). D'autres définitions de zones sensibles défectueuses peuvent être aisément envisagées par l'homme du métier.

Les signaux obtenus à partir de zones sensibles comprenant de mélanges de récepteurs non identiques permettent d'obtenir davantage d'informations non redondantes sur les constituants d'un échantillon que les signaux obtenus à partir de zones sensibles ne comprenant chacune que des récepteurs identiques. Avantageusement, cette non linéarité des signaux obtenu à l'aide des capteurs selon l'invention peut être atteinte à l'aide d'un nombre limité de récepteurs différents, ce qui est source d'économie.

Dans un autre mode de réalisation préféré, le capteur selon l'invention comprend au moins autant de zones sensibles comprenant un seul type de récepteur, qu'il y a de récepteurs différents dans le capteur, chacun des récepteurs différents du capteur étant respectivement compris dans chacune des zones sensibles comprenant un seul type de récepteur.

Les récepteurs sont fixés sur le support selon l'invention par toute technique adaptée. On peut en particulier envisager une simple adsorption, des interactions électrostatiques, ou un greffage covalent. A titre d'exemple, lorsque le support solide est du verre, on préfèrera utiliser des récepteurs ou des précurseurs de récepteur de type silane qui permettent de réaliser un greffage sur la surface du verre ; lorsque le support solide est en or ou comprend une partie en or, on préférera employer des précurseurs de récepteurs ou des récepteurs comportant des fonctions thiol qui se lieront aisément au métal. Il est également possible d'immobiliser les récepteurs dans des matrices polymères disposées sur la surface du support.

De préférence, la fixation des récepteurs sur le support est réalisée à partir de précurseurs de récepteurs, dont la structure ne diffère sensiblement pas du récepteur (la fonction permettant l'interaction peut notamment être protégée pour les besoins de la fabrication), ou des récepteurs eux-mêmes, si leur nature le permet.

### Signal

Par signal on entend, au sens de l'invention, un phénomène physique. Le signal est dit mesurable lorsqu'on peut déterminer sa présence ou son absence, et/ou le quantifier.

Le signal selon l'invention est généré suite à la mise en contact de l'échantillon avec au moins une zone sensible du capteur, il a pour origine l'interaction entre au moins un récepteur présent au sein de cette zone sensible et au moins un constituant de l'échantillon. L'interaction correspond généralement à la formation d'une liaison entre au moins un récepteur et au moins un constituant de l'échantillon, la liaison pouvant être forte ou faible et sa réversibilité possible. Selon l'invention, on mesure un signal émis pour chaque zone sensible dans son ensemble. Toutefois, si nécessaire, dans un mode de réalisation préféré de l'invention, on mesure les signaux émis par l'ensemble des zones sensibles.

Le signal est mesurable en point final ou en temps réel.

De préférence, le signal selon l'invention est mesurable en temps réel. L'expression en « temps réel » signifie que le signal est produit et mesuré essentiellement au moment où l'interaction entre le récepteur et le constituant de l'échantillon a lieu. Avantageusement, la mesure en temps réel du signal fourni est un paramètre additionnel, qui augmente encore l'information fournie par le capteur selon l'invention en ajoutant une dimension temporelle et non linéaire aux signaux.

De préférence également, le signal mesurable est de type massique, mécanique, acoustique, électrique ou optique. Selon la nature du signal selon l'invention, on pourra notamment le mesurer par microscopie optique, par microscopie à fluorescence, par microscopie confocale, par résonance plasmonique de surface, par miroir résonnant, par mesure d'impédance, par microbalance à quartz, par poutre flexible, ou par mesure d'absorption de lumière.

De préférence le capteur permettra de détecter directement le signal. Ainsi par exemple lorsque le signal est mesurable par résonance plasmonique de surface, un moyen de détection adapté correspond aux systèmes de lecteur optique de résonance de plasmons de surface. Ces systèmes sont connus, ils combinent généralement une source lumineuse, par exemple de type LED, afin de provoquer une excitation plasmonique et une caméra CCD pour enregistrer le signal produit par la résonance plasmonique. A ce titre, on préfère tout particulièrement que le signal selon l'invention soit suivi en mode imagerie. Comme l'homme du métier le sait bien, le mode imagerie consiste à suivre les variations de signal de tous les pixels constituant l'image de la caméra CCD utilisée, alors que le mode multipoint consiste lui à définir, ou à prédéfinir, des régions de l'image, c'est-à-dire un ensemble de pixels, dont la moyenne des signaux obtenus reflète un signal moyen de la région de l'image choisie.

### Support

Le support selon l'invention est constitué d'un matériau adapté à la mesure du signal.

De préférence, dans le capteur selon l'invention, les récepteurs sont fixés sur un support transduisant le signal généré par l'interaction d'un constituant du milieu avec une zone sensible, c'est-à-dire relayant le signal généré par un signal d'une autre nature qui est représentatif de celui-ci.

Lorsque le capteur est destiné à une analyse de l'échantillon par résonance plasmonique de surface, le support solide est approprié à une excitation plasmonique et à la propagation d'une onde évanescente à sa surface. Avantageusement le support sera un matériau transparent, par exemple du verre, recouvert d'une couche métallique, notamment d'or, en particulier d'épaisseur 10 à 100 nm, et plus particulièrement d'épaisseur 50 nm.

Le support selon l'invention peut notamment être constitué de verre ; de silicium ; de matériau polymère organique, en particulier de type acrylate, PDMS, COC, PEEK, nitrocellulose ; de matériau métallique, en particulier en argent, en or, ou en platine ; de matériau conducteur carboné, en particulier de type carbone vitreux, graphite, graphène, nanostructure carbonée, ou diamant ; ou d'une combinaison d'au moins deux de ces matériaux.

### Echantillon

L'échantillon selon l'invention peut être de tout type. De préférence, il s'agit d'un fluide et plus préférablement d'un échantillon de milieu liquide ou de milieu gazeux.

Il peut s'agir en particulier :
- d'un échantillon biologique, comme par exemple un échantillon de sang, de plasma, de sérum, de liquide céphalorachidien, d'urine, de selles, de liquide synovial, de sperme, de sécrétions vaginales, de sécrétions buccales, de prélèvements respiratoires, provenant en particulier des poumons, du nez, ou de la gorge, de pus, de liquides d'ascite, ou de prélèvements de sérosités cutanées ou conjonctivales ;
- d'un échantillon alimentaire, éventuellement mis en suspension, provenant notamment d'aliments, qui peuvent être crus, cuits ou cuisinés, d'ingrédients alimentaires, d'épices, de plats cuisinés, ou de boissons ;
- d'un échantillon d'eau, par exemple issu d'installations de traitement ou de distribution de l'eau ;
- d'un échantillon de sol ;
- d'un échantillon de gaz, d'air, comme par exemple un échantillon d'air ambiant ou issu d'un système de climatisation ou d'air expiré.

L'échantillon peut avoir subi au moins un traitement préalable. Le traitement peut être physique, chimique ou biologique, il peut notamment s'agir d'une extraction, d'une filtration, d'une dilution, ou d'une concentration, il peut également s'agir d'une solubilisation, d'un broyage, d'une vaporisation ou d'une mise en suspension notamment dans le cas d'un échantillon initialement solide. Le traitement peut également correspondre à l'ajout d'au moins un marqueur de signal dans le prélèvement, c'est à dire un composé émettant un signal additionnel ou susceptible de permettre, de favoriser ou d'exalter la production d'un signal par les zones sensibles. Le marqueur de signal peut se lier aux constituants de l'échantillon, il peut s'agir par exemple d'une molécule fluorescente, luminescente, ou radioactive.

Le traitement peut également correspondre à l'ajout d'un témoin interne, c'est à dire un composé dont on connait la concentration et/ou la constitution, dans l'échantillon.

Selon l'utilisation et le procédé tel que défini ci-dessus, les signaux émis par la ou les zones sensibles, mises au contact de l'échantillon, sont préférentiellement mesurés ou enregistré, notamment en fonction du temps de contact entre l'échantillon et le capteur selon l'invention.

### Cible

La cible selon l'invention peut être de tout type. De préférence il peut s'agir d'une bactérie, d'une cellule eucaryote, d'un virus, d'une protéine, d'un lipide, d'un sucre, d'un acide nucléique, de molécules organiques volatiles ou non, ou de composés inorganiques, tels des métaux. Sur le plan fonctionnel, la cible peut notamment être un médicament, une hormone, une cytokine, ou encore un récepteur cellulaire.

L'affinité, que l'on considérera ici synonyme d'avidité, de la zone sensible pour la cible est évaluée à partir du signal émis par la zone sensible. Par exemple, dans le cas où l'intensité du signal est fonction de l'affinité de la zone sensible pour la cible, un signal fort sera indicatif d'une forte affinité tandis qu'un signal faible sera indicatif d'une faible affinité, ce qui constitue les deux principales affinités recherchées.

En effet, on pourra rechercher des structures tridimensionnelles ayant une forte affinité pour au moins une première cible tout en ne se liant pas à au moins une deuxième cible.

### Analyse

L'analyse selon l'invention peut notamment viser à détecter et/ou quantifier un ou plusieurs constituants de l'échantillon. Par ailleurs, l'analyse selon l'invention peut également viser à qualifier, à catégoriser ou à classer les échantillons selon deux ou plusieurs classes prédéfinies, par exemple classer un échantillon d'origine biologique comme étant sain ou malin, ou encore selon le type de pathologie, telle qu'un cancer, qu'il représente, un échantillon d'origine alimentaire comme étant conforme ou non conforme. Dans ce cadre, on pourra procéder par comparaison à partir d'échantillons témoins, qui comprendront par exemple un constituant à l'état pur, ou qui seront représentatifs des classes à définir.

Ainsi, dans un mode de réalisation préféré du procédé selon l'invention :
- on met en contact un capteur tel que défini ci-dessus avec l'échantillon ;
- on mesure les signaux émis par les zones sensibles du capteur ;
- on compare les signaux mesurés à l'étape précédente aux signaux générés indépendamment par les zones sensibles du même capteur, ou d'un deuxième capteur similaire, consécutivement à une mise en contact avec au moins un échantillon témoin ;
- on qualifie l'échantillon analysé par rapport à au moins un échantillon témoin.

En outre, l'analyse selon l'invention peut également viser à qualifier, à catégoriser ou à classer des échantillons selon deux ou plusieurs classes non-prédéfinies. Ainsi, dans un autre mode de réalisation préféré du procédé selon l'invention :
- on met en contact un capteur tel que défini ci-dessus, ou une pluralité de capteurs similaires tels que définis ci-dessus, avec une pluralité d'échantillons ;
- on mesure les signaux émis par les zones sensibles du ou des capteurs pour chaque échantillon ;
- on compare entre eux les signaux mesurés pour chaque échantillon ;
- on catégorise chaque échantillon par rapport à l'ensemble des échantillons analysés.

Les étapes de comparaison puis de qualification définies ci-dessus peuvent nécessiter une étape d'apprentissage supervisée ou non supervisé. Cet apprentissage peut être aisément mis en œuvre par l'homme du métier du domaine des langues et des nez électroniques, qui peut notamment s'appuyer sur des techniques classiques telles que notamment décrite par Jain et al. (2000) IEEE Transactions on Pattern Analysis and Machine Intelligence 22. 4-37. A titre d'exemple, on peut citer la décomposition en composantes principales (*PCA, Principal Component Analysis*)*,* les réseaux de neurones artificiels (Turner *&* Magan, *op. cit.*), ou la séparation par machines à vecteurs supports (*SVM, Support Vector Machines*), notamment décrite par Cortes & Vapnik (1995) Machine Learning 20:273-297) et qui a été récemment utilisée avec succès pour les puces à ADN, comme cela est décrit par Brown et al. (2000) Proc. Natl. Acad. Sci. USA 97:262-267.

### Structure tridimensionnelle

Comme on l'entend ici, une « structure tridimensionnelle » selon l'invention est en particulier une particule, plus particulièrement une nanoparticule, c'est-à-dire une particule ayant au moins une dimension nanométrique, notamment de 1 nm à 999 nm, ou une microparticule, c'est-à-dire une particule ayant au moins une dimension micrométrique, notamment de 1 µm à 999 µm. Une structure tridimensionnelle selon l'invention peut notamment être un nanotube de carbone, du graphène, un dendrimère, une vésicule, une micelle, un liposome, un polymère, un nanocristal, un nanofil, des particules magnétiques ou encore des particules poreuses. De telles structures tridimensionnelles, bien connues de l'homme du métier, sont notamment utiles à titre de médicament, par exemple pour se lier à des cibles pathologiques.

La structure tridimmensionnelle est revêtue de récepteurs selon l'invention de manière à pouvoir interagir avec au moins une cible. De préférence, la structure tridimensionnelle selon l'invention est revêtue d'un mélange de récepteurs dont l'assemblage, notamment de type combinatoire, aboutit à une construction moléculaire qui interagit avec la cible, tels que ceux décrits dans Ojeda et al. (2007) Carbohydrate Research 342:448-459 ; Di Gianvincenzo et al. (2010) Bioorganic & Medicinal Chemistry Letters 20:2718-2721 ; Bresee et al. (2010) Chem. Commun. 46:7516-7518 ; Bresee et al. (2011) Small 7:2027-2031 ; ou encore Wolfenden & Cloninger, (2006) Bioconjugate Chem. 17:958-966.

L'invention sera davantage explicitée à l'aide des figures et des exemples, non limitatifs, qui suivent.

### Description des figures

Figure 1

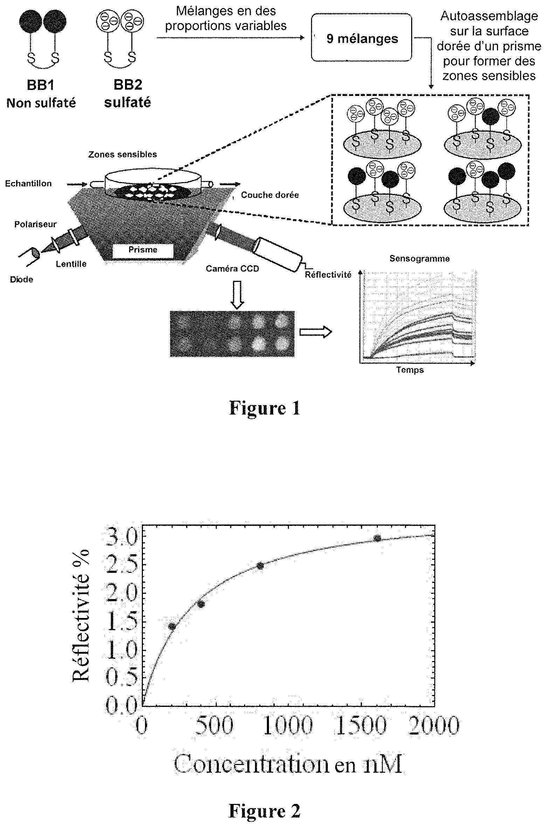

   La figure 1 décrit l'obtention et l'utilisation d'un capteur selon l'invention. Brièvement, des dépôts de neuf mélanges de lactose (BB 1) et de lactose sulfaté (BB 2) dans des proportions différentes (0, 10, 20, 30, 50, 70, 80, 90 et 100% de BB 1) sont réalisés sur des portions d'un support constitué d'un prisme recouvert d'or pour former des zones sensibles. Le capteur ainsi constitué est placé dans une cellule permettant de faire circuler différentes solutions à mettre en contact avec le capteur. La réflectivité du capteur est mesurée sous forme de sensogrammes obtenus par imagerie en résonance plasmonique de surface (SPRi) à l'aide d'une diode émettrice et d'une caméra CCD.
Figure 2
   La figure 2 représente l'évolution de la réflectivité du capteur de l'invention (axe des ordonnées, en %) mesurée par imagerie en résonance plasmonique de surface en fonction de la concentration de lectine d'*Erythrina cristagalli* (ECL) (axe des abscisses, en nM) mise en présence du capteur.
Figure 3

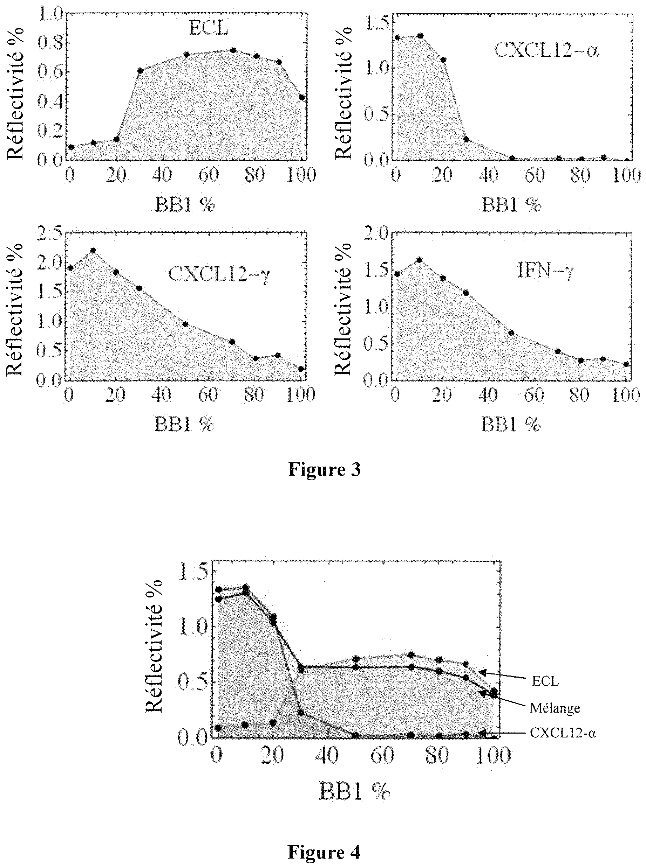

   La figure 3 représente les différents profils d'évolution continus obtenus respectivement pour ECL (200 nM), CXCL12-a (100 nM), CXCL12-y (100 nM) et IFN-γ (25 nM) avec le capteur de l'invention. Ces profils représentant la réflectivité (axe des abscisses, en %) en fonction de la proportion de BB 1 contenue dans les portions de support décrites dans la figure 1.
Figure 4
   La figure 4 représente le profil d'évolution continu d'un mélange ECL + CXCL12-a et ceux correspondant respectivement à ECL et CXCL12-a seuls.
Figure 5

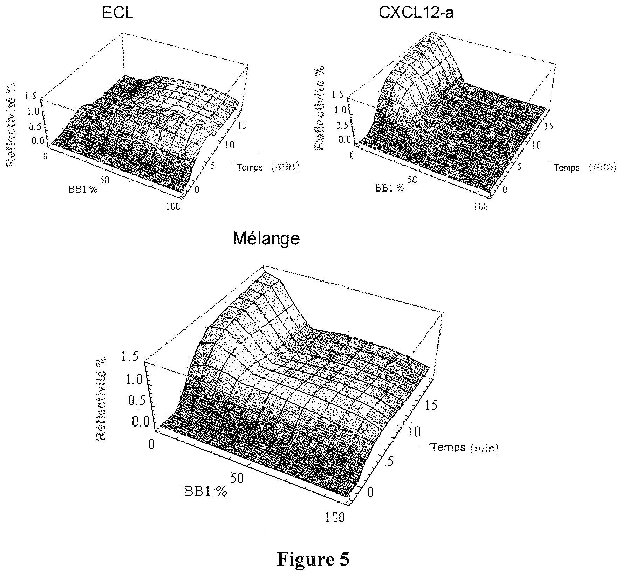

   La figure 5 représente des modèles 3D des profils d'évolution continus, en fonction du temps de contact avec le capteur, obtenus respectivement pour ECL seul (en haut à gauche), pour CXCL12-a seul (en haut à droite) et pour un mélange ECL + CXCL12-a.
Figure 6

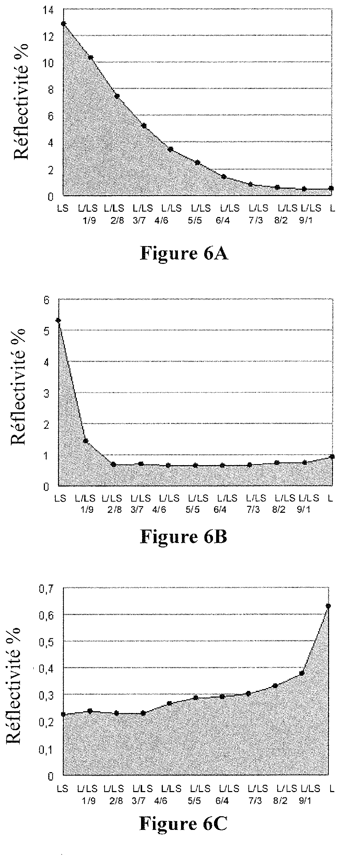

   La figure 6 représente des profils d'évolution continus obtenus pour des mélanges alimentaires : à savoir le lait de soja (Figure 6A), le lait de vache (Figure 6B) et le lait de riz (Figure 6C).
Figure 7

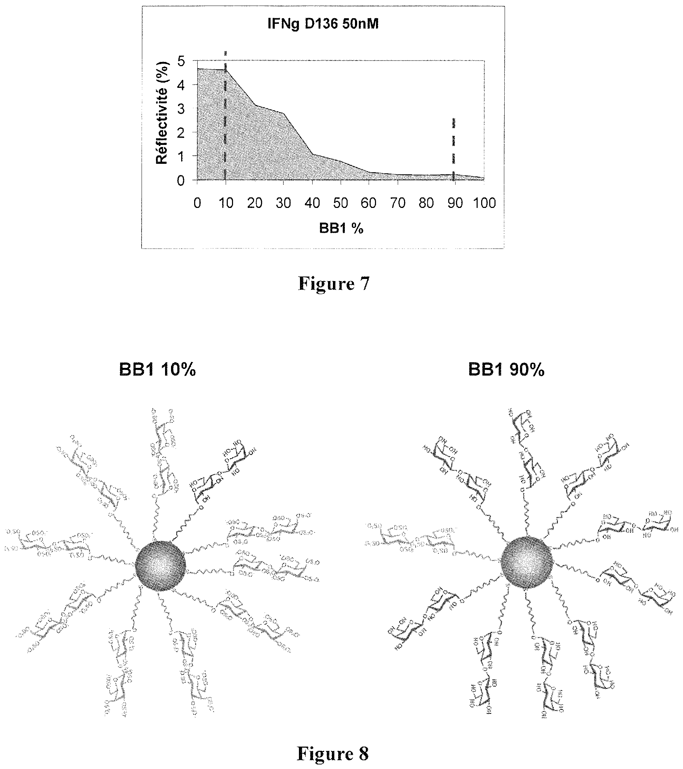

   La figure 7 représente la réflectivité mesurée par imagerie en résonance plasmonique de surface (axe des ordonnées, en %) pour les zones sensibles d'un capteur selon l'invention chacune représentatives d'un mélanges de lactose (BB 1) et de lactose sulfaté (BB 2) dans des proportions différentes (0, 10, 20, 30, 50, 70, 80, 90 et 100% de BB 1) (axe des abscisses) en présence d'interféron gamma D136 (IFNg D136).
Figure 8
   La figure 8 représente une nanoparticule recouverte d'un mélange BB 1 10%/BB 2 90% (à gauche) et une nanoparticule recouverte d'un mélange BB 1 90%/BB 2 10% (à droite).
Figure 9

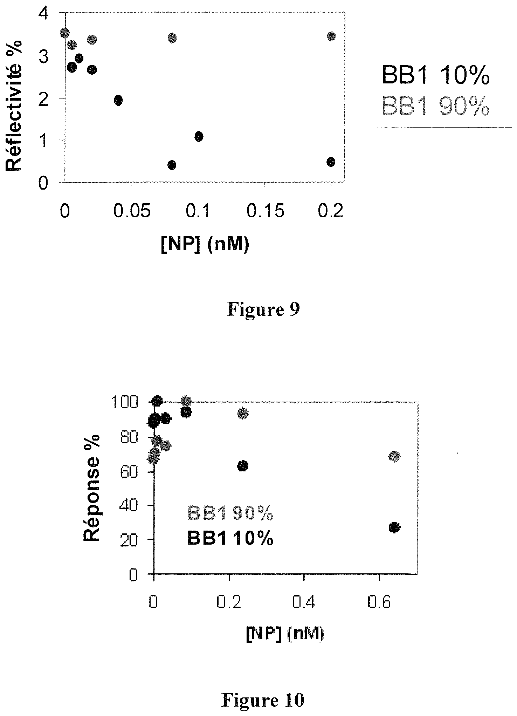

   La figure 9 représente le résultat de la mesure par SPRi sur le plot BB 1 à 10% de l'interaction de l'IFNgD136 mis en présence des nanoparticules d'or fonctionnalisées avec deux types de revêtements : BB 1 à 10% ou BB 1 à 90%. Cet effet est dépendant de la concentration en nanoparticules.
Figure 10
   La figure 10 représente le dosage de l'IFNgD136 par analyse ELISA sur un anticorps anti interferon. L'IFNgD136 est incubé préalablement avec des nanoparticules d'or fonctionnalisées avec deux types de revêtements : BB 1 à 10% ou BB 1 à 90%. La concentration du surnageant en IFNgD136 est ensuite évaluée par test ELISA.

### Exemples

### Exemple 1 : capteur dont les zones sensibles sont constituées de mélanges de 2 récepteurs

### 1. Fabrication du capteur

Deux molécules simples ont été utilisées comme briques de construction (BB) pour construire un capteur comprenant un support accommodant plusieurs zones sensibles combinant des proportions variables de récepteurs : le lactose (BB 1) et le lactose sulfaté (BB 2). Neuf mélanges avec des rapports [BB1]/([BB1]+[BB2]) de 0, 10, 20, 30, 50, 70, 80, 90 et 100% ont été préparés, la concentration totale étant constante à 20 µM. Les mélanges ont ensuite été déposés sur un support formé par la surface en or d'un prisme utilisable pour l'imagerie en résonance plasmonique de surface (SPRi) et ont été maintenus en contact avec la surface du prisme sur la nuit. La puce ainsi constituée a ensuite été lavée à l'éthanol puis séchée sous un flux de N₂.

En conséquence, après auto-assemblage un capteur comprenant neuf zones sensibles ayant chacune des proportions différentes de BB 1 et BB 2 a pu être obtenu, comme cela est indiqué dans la **Figure 1**. Ce capteur a ensuite été utilisé pour analyser des milieux comprenant une seule protéine par SPRi.

### 2. Analyse de milieux témoins contenant une seule protéine

L'analyse des milieux protéiques a été réalisée dans une cellule en Teflon de 10 µl, connectée à un dégazeur et à une pompe péristaltique. 500 µl de milieu protéique ont été injectés. La solution tampon de travail utilisée comprenait 10 mM HEPES, 150 mM NaCl, 0,005% Tween, 20, 2 mM MgCl₂, pH 7,4, et a été filtrée et dégazée avant utilisation. Toutes les expériences ont été conduites à température ambiante avec un flux de 100µL/min. Avant chaque injection de protéine, de l'albumine de sérum bovin a été utilisée pour bloquer la surface d'or nue, afin d'éviter les interactions non spécifiques.

Quatre protéines se liant aux sucres ont été testées : la lectine d'*Erythrina cristagalli* (ECL), les isoformes α et γ de la chimiokine CXCL12 et la cytokine pro-inflamatoire interféron-γ (IFN-γ). Ces protéines peuvent être classées en deux groupes en fonction de leur capacité à se lier à des chaînes osidiques : ECL est une lectine qui se lie au galactose, alors que les deux isoformes de CXCL12 ainsi que l'IFN-γ se lient à l'héparane sulfate.

En pratique, ECL a été utilisée en premier pour tester le capteur à plusieurs concentrations (200 nM, 400 nM, 800 nM et 1.6 µM). Une courbe de calibration (voir la **Figure 2**) a été établie. Cette courbe présente un profile d'adsorption de type Langmuir, ce qui confirme que le système fonctionne correctement en tant que capteur avec un K_{D} = 300+/- 150nM.

Lors de tests préliminaires deux concentrations différentes ont été utilisées pour les autres protéines (100 et 200 nM pour les isoformes de CXCL12, 25 et 50 nM pour l'IFN-γ), afin de choisir les concentrations auxquelles les signaux émis sont comparables. Finalement, les concentrations qui ont été choisies sont les suivantes : ECL 200 nM, CXCL12-a 100 nM, CXCL12-y 100 nM et IFN-γ 25 nM. Il convient également de noter qu'après chaque analyse de protéine, le capteur a été régénéré par des solutions appropriées. Dans ce but, différentes solutions ont été testées afin d'identifier la solution adaptée à chaque protéine. Ont ainsi été retenues des solutions à 0,02 M NaOH pour ECL, 1 M NaCl pour CXCL12-a et 1% SDS for CXCL12-y et IFN- y, car elles permettent une régénération complète du capteur sans provoquer de dégâts.

Les protéines pures aux concentrations choisies ont été injectées successivement sur les capteurs, les dépôts de mélanges sur le support du capteur s'allumant alors à différents niveaux de gris. Ces images ont été enregistrées puis ensuite converties en séries de sensogrammes.

De manière surprenante, les inventeurs ont observé que pour une protéine donnée la réflectivité était dépendante de la composition en BB des zones sensibles, comme cela est visible dans la **Figure 3**. On observe notamment que la réponse des différents mélanges de BB n'est pas la simple addition linéaire de la réponse des récepteurs purs. Ce comportement non linéaire justifie donc *a posteriori* l'utilisation de différentes zones sensibles avec des proportions variables de chacun des récepteurs puisque la réponse de chaque zone sensible est porteuse d'une information supplémentaire. De plus, on observe que pour une zone sensible donnée, l'intensité de la réponse dépend de la protéine injectée, ce qui indique que le capteur répond différemment à chaque protéine.

Afin d'illustrer le comportement individuel de chaque protéine, les valeurs de réflectivité mesurées une minute avant la fin de l'injection de protéine ont été représentées en fonction de la proportion de BB 1 dans les mélanges **(****Figure 3****)**. De manière intéressante, un profil distinctif sous la forme d'un profil d'évolution continu à pu être interpolé pour chaque protéine. Les inventeurs ont pu mettre en évidence que pour une protéine donnée le profil était essentiellement maintenu quelle que soit la concentration de la protéine, l'intensité du signal étant quant à elle bien sûr variable en fonction de la concentration.

En conséquence, de tels capteurs pourraient être utilisés non seulement pour différencier et identifier des protéines mais également à des fins de quantification. De plus, le comportement continu de la réponse de ces capteurs à une protéine procure un avantage significatif et important par rapport aux ensembles de données obtenus avec les capteurs de nez et de langue électroniques traditionnels. En effet, comme pour chacun des profils d'évolution continue les signaux d'un récepteur sont corrélés aux autres, les signaux anormaux peuvent être exclus, ce qui permet de réaliser des identifications d'analytes plus précises et plus justes.

De manière détaillée, le profil d'ECL, avec un signal maximum à 70% de BB 1, est complètement différent des autres avec des signaux maximums à 10% de BB 1, indiquant que, comme attendu, ECL a une affinité supérieure pour les zones sensibles de support riches en lactose. Au contraire, les protéines se liant à l'héparane sulfate ont une affinité supérieure pour les zones sensibles riches en lactose sulfaté BB 2. En conséquence, ECL peut être facilement distingué des autres. De manière plus intéressante, l'isoforme α de CXCL12 donne un profil d'évolution continu relativement différent de ceux obtenus pour CXCL12-y ou l'IFN-γ. En effet, pour CXCL12-a la réflectivité est quasi nulle lorsque la proportion de BB 1 est de 50% ou plus, alors que pour CXCL12- γ ou l'IFN-γ elle est beaucoup plus élevée. Une analyse plus poussée du profil d'évolution continu représenté dans la **Figure 3** révèle qu'à la même concentration CXCL12-y possède une affinité beaucoup plus élevée pour les zones sensibles que CXCL12-a. Ainsi, alors qu'avec CXCL12-a à 100 nM, aucune zone sensible du capteur ne présente une réflectivité supérieure à 1,35 %, un signal de 2,30 % est atteint avec CXCL12- y.

Si l'on peut aisément comprendre que l'on puisse distinguer ECL des autres protéines à l'aide du capteur, il est à noter que les deux isoformes de CXCL12, qui sont identiques dans leurs régions 1-68, sont mieux distinguées que CXCL12-y par rapport à IFN-γ.

Toutefois, avec cette première génération de capteur comprenant 9 zones sensibles ayant des proportions différentes de 2 récepteurs, il n'est pas possible de distinguer CXCL12-γ et IFN-γ malgré la différence d'intensité de signal. A cet égard, les inventeurs prévoient que des capteurs préparés à partir de récepteurs additionnels afin de générer plus de diversité au niveau des zones sensibles, devraient permettre de distinguer des protéines de liaison à l'héparane sulfate avec des topologies de charges similaires mais non identiques.

### 3. Application à l'analyse de milieux complexes

Les principales applications de la technologie des nez et langue électroniques se trouvent dans le contrôle et l'analyse de milieux complexes. Afin de tester l'efficacité du capteur ci-dessus dans l'analyse de milieu, les inventeurs l'ont testé avec un mélange de deux protéines : ECL (200 nM) et CXCL12-a (100 nM) (Mélange 1) ainsi que des mélanges alimentaires tels que du lait de soja, du lait de vache ou du lait de riz.

Le profil d'évolution continu obtenu est présenté dans la **Figure 4**, accompagné des profils obtenus pour les deux protéines pures pour comparaison. Ce résultat initial démontre que le capteur est sensible au mélange et qu'il est capable de distinguer le mélange des protéines pures. En fait, on observe même que le profil d'un mélange de deux protéines est proche d'une addition simple des profils des protéines pures. Ceci suggère qu'il n'existe quasiment aucune interaction coopérative entre les protéines adsorbées sur les zones sensibles du capteur. Le principal avantage de cette propriété est de rendre possible la détection et la quantification de mélanges à partir des profils respectifs des composants individuels du mélange par simple décomposition linéaire.

Par ailleurs, il serait avantageux de prendre avantage des cinétiques d'adsorption et de désorption en temps réel obtenus par SPRi. Cette information supplémentaire pourrait rajouter une autre façon de distinguer les différentes protéines, en complément des profils d'évolution continu. A titre d'illustration, la **Figure 5** présente l'évolution temporelle du profil de reconnaissance du mélange ECL + CXCL12-a en trois dimensions.

En outre, afin de mieux comparer la réponse de la langue électronique vis-à-vis de trois types d'échantillons alimentaires, du lait de soja, du lait de vache ou du lait de riz, un profil a été réalisé pour chaque échantillon. Ce profil représente la réflectivité après 6 min de rinçage en fonction des rapports lactose/lactose sulfaté (L/LS) des zones sensibles **(****Figure 6**). On peut confirmer très facilement que pour le lait de soja (**Figure 6A**), il y a une forte affinité avec plusieurs zones sensibles riches en lactose sulfaté incluant le lactose sulfaté pur, 90% LS, 80% LS, 70% LS, 60% LS, 50% LS. En revanche, pour le lait UHT (**Figure 6B**), il y a une forte affinité seulement avec 2 zones sensibles, lactose sulfaté pur et 90% LS. Ceci montre que la langue électronique est capable de différencier ces 2 produits. S'agissant du lait de riz (**Figure 6C**), le profil est complètement différent car la plus forte affinité est obtenue avec la zone sensible composée de lactose pur. Ces résultats montrent que la langue électronique est efficace pour analyser et différencier des échantillons complexes. De plus, le profil obtenu pour chaque échantillon peut être considéré comme une signature pour leur identification.

### Exemple 2 : méthode de sélection de surfaces combinatoires adaptées

L'utilisation de nanoparticules (NP) décorées comme agent thérapeutique est un sujet très exploré. Plusieurs auteurs ont ainsi décrit des systèmes ayant une certaine efficacité. Parmi les revêtements utilisés, il est possible de citer :(i) des ligands spécifiques, comme des anticorps par exemple, qui interagiront directement avec leur cible, molécule à molécule, ou,(ii) développés plus récemment, des revêtements composés d'une petite molécule (Bowman et al. (2008) J. Am. Chem. Soc. 130:6896-6897 ; Baram-Pinto et al. (2009) Bioconjugate Chem. 20:1497-1502 ; Baram-Pinto et al. (2010) Small 6:1044-1050 ; Rele et al. (2005) J. Am. Chem. Soc. 127:10132-10133) ou d'un assemblage de petites molécules en elles-mêmes individuellement sans propriétés biologiques très définies (Ojeda et al. (2007) Carbohydrate Research 342:448-459 ; Di Gianvincenzo et al. (2010) Bioorganic & Medicinal Chemistry Letters 20:2718-2721 ; Bresee et al. (2010) Chem. Commun. 46:7516-7518 ; Bresee et al. (2011) Small 7:2027-2031 ; Wolfenden & Cloninger, (2006) Bioconjugate Chem. 17:958-966) mais générant des propriétés spécifiques lorsque déposées sur la surface des nanoparticules.

Ces derniers assemblages sont classiquement réalisés de façon combinatoire : des ensembles définis de briques de base sont mélangés puis assemblés sur la nanoparticule. Le choix de ces mélanges n'est pas forcément rationnel puisque les structures adéquates sont finalement identifiées en criblant l'activité de chaque type de NP.

Cela signifie donc que pour trouver des revêtements actifs sur une base de banques de composés n'ayant pas forcément d'activité biologique, il faudra fabriquer un grand nombre de NP munis de revêtements issus de mélanges combinatoires. Le criblage de l'activité biologique de ces NP permettra, éventuellement, de détecter quels sont les mélanges actifs. Il résulte de cela un très faible rapport entre le nombre de NP différentes préparées et le nombre de NP actives.

Afin de mieux cibler ces mélanges et donc de diminuer le nombre de NP non actives fabriquées, il serait avantageux de pouvoir évaluer rapidement l'activité des mélanges des briques de base formant récepteur sous un format plus propice à l'analyse rapide à haut débit.

Dans ce cadre, les inventeurs ont montré, de façon surprenante, que l'activité d'une surface composée d'un mélange de récepteurs sur un support à 2 dimensions (2D) reste très proche de celle d'une nanoparticule revêtue du même mélange.

Ainsi, les inventeurs proposent d'évaluer l'activité de nanoparticules, ou plus généralement de structures tridimensionnelles, portant à leur surface un mélange de composés formant récepteur grâce à un test sur un capteur 2D portant un certains nombre de zones sensibles, par exemple sous forme de spots, qui sont chacune représentative d'un mélange en deux proportions particulières des composés initiaux. La cible, qui peut être une protéine ou un microorganisme par exemple, est alors mise en contact avec ces capteurs et les interactions sont mesurées, par SPRi par exemple. L'intensité des interactions mesurées entre la cible et les différentes zones sensibles sont indicatives des compositions de mélange à privilégier pour la fabrication du revêtement des structures tridimensionnelles, telles que les NP. De même, si l'on préfère éviter une interaction, on choisira de préférence une composition de mélange proche de celle d'une zone sensible présentant pas ou peu d'interactions avec la cible définie.

Les inventeurs proposent donc d'associer à la fabrication combinatoire de revêtements de structures tridimensionnelles, notamment du type nano ou micro-objet, telles que des NP, des dendrimères, ou des liposomes, une étape de criblage 2D à l'aide d'un capteur selon l'invention, très bien adaptée a l'évaluation rapide des propriétés des surfaces. La conservation des propriétés 2D vers 3D permet ainsi de présélectionner des surfaces actives, ce qui rend le développement de futurs médicaments beaucoup plus rapide et moins couteux.

### Description du principe :

1- Un capteur est tout d'abord construit comme indiqué dans l'Exemple 1 en préparant des zones sensibles constituées chacune d'un mélange avec une proportion propre des briques de base formant récepteur, à savoir neuf mélanges avec des rapports [BB1]/([BB1]+[BB2]) de 0, 10, 20, 30, 50, 70, 80, 90 et 100%, la concentration totale étant constante à 20 µM, BB 1 représentant le lactose et BB 2 le lactose sulfaté.
2- L'affinité des zones sensible pour la cible, ici l'interféron gamma D136 (IFNg D136) est alors mesurée, dans le cas présent par SPRi selon les modalités de l'Exemple 1.
3- On sélectionne la ou les zones sensibles offrant la meilleure affinité. Dans le cas présent, la zone sensible pertinente pour la protéine IFNg D136 est constituée d'un mélange 10% BB 1/90% BB 2 (**Figure 7**). La zone sensible correspondant au mélange 90% BB 1/10% BB 2 a été choisi comme contrôle négatif.
4- On prépare des NP, particule d'or, revêtue de mélanges similaires à ceux identifiés (**Figure 8**). La taille des nanoparticules est de 20 nm.
5- On vérifie l'affinité des NP préparées pour la cible à l'aide de tests biochimiques classiques, ici par SPRi (Figure 9) ou par ELISA (Figure 10).

En SPRi on observe bien que les NP avec le revêtement pertinent (BB1 10%) provoquent une diminution de signal qui est dépendant de la concentration de NP ; en revanche, les NP contrôle négatif (BB1 90%) ne provoquent pas de variation de signal du tout.

Les résultats de l'ELISA confirment que l'affinité pour une cible d'une zone sensible, constituée d'un mélange de récepteurs sur un support à 2 dimensions, reste très proche de celle d'une nanoparticule revêtue du même mélange.

Par ailleurs, le même type de procédé, de l'étape 1 à 5 peut être réalisé sur plusieurs cibles ; par exemple si l'on désire que la particule fixe une première cible A sans fixer une deuxième cible B.

## Revendications

1. Capteur pour langue ou nez électronique pour analyser un échantillon ou pour détecter au moins une cible, comprenant un support sur une surface duquel se trouve une pluralité de zones sensibles comprenant chacune au moins un récepteur, chaque zone sensible pouvant émettre un signal mesurable généré par l'interaction d'au moins un constituant de l'échantillon ou d'au moins une cible avec au moins un récepteur, **caractérisé en ce qu'**il comprend au moins trois zones sensibles qui diffèrent entre elles par leurs compositions respectives en récepteur, l'une au moins des zones sensibles comprenant un mélange d'au moins deux récepteurs différents, les deux autres zones sensibles comprenant chacune au moins un des deux récepteurs.

2. Capteur pour langue ou nez électronique selon la revendication 1, comprenant une pluralité de zones sensibles qui comprennent chacune un mélange d'au moins deux récepteurs différents, les récepteurs différents étant les mêmes dans chaque mélange, et qui diffèrent entre elles par les proportions respectives des récepteurs différents qu'elles comprennent.

3. Capteur pour langue ou nez électronique selon la revendication 1 ou 2, dans lequel l'une au moins des zones sensibles comprend un mélange d'au moins trois récepteurs différents.

4. Capteur pour langue ou nez électronique selon l'une des revendications 1 à 3, comprenant une pluralité de zones sensibles qui comprennent chacune un mélange d'au moins trois récepteurs différents, les récepteurs différents étant les mêmes dans chaque mélange, et qui diffèrent entre elles par les proportions respectives des récepteurs différents qu'elles comprennent.

5. Capteur pour langue ou nez électronique selon l'une des revendications 1 à 4, comprenant au moins autant de zones sensibles comprenant un seul type de récepteur, qu'il y a de récepteurs différents dans le capteur, chacun des récepteurs différents du capteur étant respectivement compris dans chacune des zones sensibles comprenant un seul type de récepteur.

6. Capteur pour langue ou nez électronique selon l'une des revendications 1 à 5, comprenant un nombre de zones sensibles suffisant pour que le profil des signaux émis par l'ensemble des zones permette d'identifier la présence d'au moins une zone sensible défectueuse.

7. Utilisation d'un capteur tel que défini dans l'une des revendications 1 à 6, pour analyser un échantillon.

8. Procédé pour analyser un échantillon, dans lequel :
- on met en contact un capteur tel que défini dans l'une des revendications 1 à 6 avec l'échantillon ;
- on mesure les signaux émis par les zones sensibles du capteur ;
- on compare les signaux mesurés à l'étape précédente aux signaux générés indépendamment par les zones sensibles du même capteur, ou d'un deuxième capteur similaire, consécutivement à une mise en contact avec au moins un autre échantillon ;
- on qualifie l'échantillon analysé.

9. Procédé selon la revendication 8, dans lequel :
- on met en contact un capteur tel que défini ci-dessus avec l'échantillon ;
- on mesure les signaux émis par les zones sensibles du capteur ;
- on compare les signaux mesurés à l'étape précédente aux signaux générés indépendamment par les zones sensibles du même capteur, ou d'un deuxième capteur similaire, consécutivement à une mise en contact avec au moins un échantillon témoin ;
- on qualifie l'échantillon analysé par rapport à au moins un échantillon témoin.

10. Procédé selon la revendication 8, dans lequel :
- on met en contact un capteur tel que défini ci-dessus, ou une pluralité de capteurs similaires tels que définis ci-dessus, avec une pluralité d'échantillons ;
- on mesure les signaux émis par les zones sensibles du ou des capteurs pour chaque échantillon ;
- on compare entre eux les signaux mesurés pour chaque échantillon ;
- on catégorise chaque échantillon par rapport à l'ensemble des échantillons analysés.

11. Procédé selon l'une des revendications 8 à 10, comprenant en outre une étape de vérification de la fonctionnalité des zones sensibles du capteur par comparaison du signal émis par une zone sensible avec celui émis par au moins deux autres zones sensibles.

12. Utilisation d'un capteur tel que défini dans l'une des revendications 1 à 6, pour définir la composition en récepteurs d'un mélange de récepteurs pour revêtir une structure tridimensionnelle destinée à interagir ou à ne pas interagir avec au moins une cible.

13. Procédé de préparation d'une structure tridimensionnelle revêtue d'un mélange de récepteurs destinée à interagir ou à ne pas interagir avec au moins une cible, comprenant :
- la mise en contact de la cible avec un capteur tel que défini dans l'une des revendications 1 à 6 ;
- l'identification d'une zone sensible du capteur émettant un signal dont l'intensité est indicative d'une affinité pour la cible similaire à l'affinité souhaitée de la structure tridimensionnelle revêtue d'un mélange de récepteurs pour la cible ;
- revêtir la structure tridimensionnelle avec un mélange de récepteurs ayant une composition en récepteurs similaire à celle de la zone sensible identifiée.

14. Procédé selon la revendication 13, comprenant en outre une étape de vérification de la fonctionnalité des zones sensibles par comparaison du signal émis de chaque zone sensible avec celui émis par au moins deux autres zones sensibles.

## Patentansprüche

1. Sensor für elektronische Zunge oder Nase, um eine Probe zu analysieren oder um mindestens ein Ziel zu erfassen, umfassend einen Träger, bei dem sich auf einer Oberfläche davon eine Vielzahl von empfindlichen Bereichen befindet, die jeweils mindestens einen Rezeptor umfassen, wobei jeder empfindliche Bereich ein messbares Signal ausstrahlen kann, das durch die Wechselwirkung mindestens eines Bestandteils der Probe oder mindestens eines Ziels mit mindestens einem Rezeptor erzeugt wird, **dadurch gekennzeichnet, dass** er mindestens drei empfindliche Bereiche umfasst, die sich voneinander in ihren jeweiligen Zusammensetzungen an Rezeptoren unterscheiden, wobei der mindestens eine der empfindlichen Bereiche ein Gemisch von mindestens zwei unterschiedlichen Rezeptoren umfasst, wobei die zwei anderen empfindlichen Bereiche jeweils mindestens einen der zwei Sensoren umfassen.

2. Sensor für elektronische Zunge oder Nase nach Anspruch 1, umfassend eine Vielzahl von empfindlichen Bereichen, die jeweils ein Gemisch von mindestens zwei unterschiedlichen Rezeptoren umfassen, wobei die unterschiedlichen Rezeptoren in jedem Gemisch die gleichen sind, und die sich durch die jeweiligen Proportionen an unterschiedlichen Rezeptoren, die sie umfassen, voneinander unterscheiden.

3. Sensor für elektronische Zunge oder Nase nach Anspruch 1 oder 2, wobei der mindestens eine der empfindlichen Bereiche ein Gemisch von mindestens drei unterschiedlichen Rezeptoren umfasst.

4. Sensor für elektronische Zunge oder Nase nach einem der Ansprüche 1 bis 3, umfassend eine Vielzahl von empfindlichen Bereichen, die jeweils ein Gemisch von mindestens drei unterschiedlichen Rezeptoren umfassen, wobei die unterschiedlichen Rezeptoren in jedem Gemisch die gleichen sind, und die sich durch die jeweiligen Proportionen an unterschiedlichen Rezeptoren, die sie umfassen, voneinander unterscheiden.

5. Sensor für elektronische Zunge oder Nase nach einem der Ansprüche 1 bis 4, umfassend mindestens ebenso viele empfindliche Bereiche, die eine einzige Art von Rezeptor umfassen, wie es unterschiedliche Rezeptoren im Sensor gibt, wobei jeder der unterschiedlichen Rezeptoren des Sensors jeweils in jedem der empfindlichen Bereiche umfasst sind, der eine einzige Art von Rezeptor umfasst.

6. Sensor für elektronische Zunge oder Nase nach einem der Ansprüche 1 bis 5, umfassend eine Anzahl an empfindlichen Bereichen, die ausreicht, damit das Profil der von der Gesamtheit der Bereiche ausgestrahlten Signale es ermöglicht, das Vorhandensein mindestens eines fehlerhaften empfindlichen Bereichs zu identifizieren.

7. Verwendung eines Sensors wie in einem der Ansprüche 1 bis 6 definiert, zum Analysieren einer Probe.

8. Verfahren zum Analysieren einer Probe, wobei:
- ein Sensor wie in einem der Ansprüche 1 bis 6 definiert mit der Probe in Kontakt gebracht wird;
- die von den empfindlichen Bereichen des Sensors ausgestrahlten Signale gemessen werden;
- die im vorangegangenen Schritt gemessenen Signale mit den Signalen verglichen werden, die unabhängig von den empfindlichen Bereichen des gleichen Sensors oder von einem zweiten, ähnlichen Sensor im Anschluss an ein Inkontaktbringen mit mindestens einer anderen Probe erzeugt werden;
- die analysierte Probe bewertet wird.

9. Verfahren nach Anspruch 8, wobei:
- ein Sensor wie vorstehend definiert mit der Probe in Kontakt gebracht wird;
- die von den empfindlichen Bereichen des Sensors ausgestrahlten Signale gemessen werden;
- die im vorangegangenen Schritt gemessenen Signale mit den Signalen verglichen werden, die unabhängig von den empfindlichen Bereichen des gleichen Sensors oder von einem zweiten, ähnlichen Sensor im Anschluss an ein Inkontaktbringen mit mindestens einer Kontrollprobe erzeugt werden;
- die analysierte Probe in Bezug auf mindestens eine Kontrollprobe bewertet wird.

10. Verfahren nach Anspruch 8, wobei:
- ein Sensor wie vorstehend definiert oder eine Vielzahl von ähnlichen Sensoren wie vorstehend definiert mit einer Vielzahl von Proben in Kontakt gebracht wird;
- die von den empfindlichen Bereichen des oder der Sensoren ausgestrahlten Signale für jede Probe gemessen werden;
- die gemessenen Signale für jede Probe untereinander verglichen werden;
- jede Probe in Bezug auf die Gesamtheit der analysierten Proben kategorisiert wird.

11. Verfahren nach einem der Ansprüche 8 bis 10, weiter umfassend einen Schritt der Überprüfung der Funktionsfähigkeit der empfindlichen Bereiche des Sensors durch Vergleich des Signals, das von einem empfindlichen Bereich ausgestrahlt wird, mit jenem, das von mindestens zwei anderen empfindlichen Bereichen ausgestrahlt wird.

12. Verwendung eines Sensors wie in einem der Ansprüche 1 bis 6 definiert, um die Zusammensetzung an Rezeptoren eines Rezeptorgemischs zu definieren, um eine dreidimensionale Struktur zu beschichten, die dazu vorgesehen ist, mit mindestens einem Ziel zu wechselwirken oder nicht zu wechselwirken.

13. Verfahren zum Herstellen einer dreidimensionalen Struktur, die mit einem Rezeptorgemisch beschichtet ist, die dazu vorgesehen ist, mit mindestens einem Ziel zu wechselwirken oder nicht zu wechselwirken, umfassend:
- das Inkontaktbringen des Ziels mit einem Sensor wie in einem der Ansprüche 1 bis 6 definiert;
- die Identifizierung eines empfindlichen Bereichs des Sensors, der ein Signal ausstrahlt, dessen Intensität auf eine Affinität für das Ziel hinweist, ähnlich der gewünschten Affinität der dreidimensionalen, mit einem Rezeptorgemisch beschichteten, Struktur für das Ziel;
- Beschichten der dreidimensionalen Struktur mit einem Rezeptorgemisch, das eine Zusammensetzung an Rezeptoren, ähnlich jener des identifizierten empfindlichen Bereichs, aufweist.

14. Verfahren nach Anspruch 13, weiter umfassend einen Schritt der Überprüfung der Funktionsfähigkeit der empfindlichen Bereiche durch Vergleich des von jedem empfindlichen Bereich ausgestrahlten Signals mit jenem, das von mindestens zwei anderen empfindlichen Bereichen ausgestrahlt wird.

## Claims

1. An electronic tongue or nose sensor for analyzing a sample or detecting at least one target, comprising a support on one surface of which is a plurality of sensitive zones, each comprising at least one receptor, it being possible for each sensitive zone to emit a measurable signal generated by the interaction of at least one constituent of the sample or of at least one target with at least one receptor, **characterized in that** it comprises at least three sensitive zones which differ from one another by virtue of their respective receptor compositions, at least one of the sensitive zones comprising a mixture of at least two different receptors, the other two sensitive zones each comprising at least one of the two receptors.

2. The electronic tongue or nose sensor as claimed in claim 1, comprising a plurality of sensitive zones which each comprise a mixture of at least two different receptors, the different receptors being the same in each mixture, and which differ from one another in terms of the respective proportions of the different receptors that they comprise.

3. The electronic tongue or nose sensor as claimed in claim 1 or 2, wherein at least one of the sensitive zones comprises a mixture of at least three different receptors.

4. The electronic tongue or nose sensor as claimed in one of claims 1 to 3, comprising a plurality of sensitive zones which each comprise a mixture of at least three different receptors, the different receptors being the same in each mixture, and which differ from one another in terms of the respective proportions of the different receptors that they comprise.

5. The electronic tongue or nose sensor as claimed in one of claims 1 to 4, comprising at least as many sensitive zones comprising a single type of receptor as there are different receptors in the sensor, each of the different receptors of the sensor being respectively included in each of the sensitive zones comprising a single type of receptor.

6. The electronic tongue or nose sensor as claimed in one of claims 1 to 5, comprising a sufficient number of sensitive zones for the profile of the signals emitted by all the zones to make it possible to identify the presence of at least one defective sensitive zone.

7. The use of a sensor as defined in one of claims 1 to 6, for analyzing a sample.

8. A process for analyzing a sample, in which:
- a sensor as defined in one of claims 1 to 6 is brought into contact with the sample;
- the signals emitted by the sensitive zones of the sensor are measured;
- the signals measured in the previous step are compared with the signals generated independently by the sensitive zones of the same sensor, or of a second similar sensor, subsequent to bringing into contact with at least one other sample;
- the sample analyzed is described.

9. The process as claimed in claim 8, in which:
- a sensor as defined above is brought into contact with the sample;
- the signals emitted by the sensitive zones of the sensor are measured;
- the signals measured in the previous step are compared with the signals generated independently by the sensitive zones of the same sensor, or of a second similar sensor, subsequent to bringing into contact with at least one control sample;
- the sample analyzed is described with respect to at least one control sample.

10. The process as claimed in claim 8, in which:
- a sensor as defined above, or a plurality of similar sensors as defined above, is brought into contact with a plurality of samples;
- the signals emitted by the sensitive zones of the sensor(s) are measured for each sample;
- the signals measured for each sample are compared with one another;
- each sample is categorized with respect to all the samples analyzed.

11. The process as claimed in one of claims 8 to 10, also comprising a step of verifying the functionality of the sensitive zones of the sensor by comparison of the signal emitted by a sensitive zone with that emitted by at least two other sensitive zones.

12. The use of a sensor as defined in one of claims 1 to 6, for defining the receptor composition of a mixture of receptors for coating a three-dimensional structure intended to interact or not to interact with at least one target.

13. A process for preparing a three-dimensional structure coated with a mixture of receptors, intended to interact or not to interact with at least one target, comprising:
- bringing the target into contact with a sensor as defined in one of claims 1 to 6;
- identifying a sensitive zone of the sensor emitting a signal of which the intensity is indicative of an affinity for the target similar to the desired affinity of the three-dimensional structure coated with a mixture of receptors for the target;
- coating the three-dimensional structure with a mixture of receptors having a receptor composition similar to that of the sensitive zone identified.

14. The process as claimed in claim 13, also comprising a step of verifying the functionality of the sensitive zones by comparison of the signal emitted by each sensitive zone with that emitted by at least two other sensitive zones.
